# EUROPÄISCHE PATENTSCHRIFT

(11) **EP 0 426 762 B1**
(45) Veröffentlichungstag und Bekanntmachung des Hinweises auf die Patenterteilung: **16.03.1994**
(21) Anmeldenummer: 89910088.7
(22) Anmeldetag: 09.09.1989
(51) Int. Cl.: C07K 1/02, C07K 1/12

(54) **VERFAHREN ZUR HERSTELLUNG VON DIPEPTIDEN MIT C-TERMINALEN NICHT-PROTEINOGENEN AMINOSÄUREN**
PROCESS FOR PRODUCING DIPEPTIDES FROM NON-PROTEINOGENIC AMINO ACIDS WITH TERMINAL CARBON ATOMS
PROCEDE DE PRODUCTION DE DIPEPTIDES A PARTIR D'AMINOACIDES NON PROTEINOGENES A TERMINAISONS C

(30) Priorität: 17.09.1988 DE 3831716
(43) Veröffentlichungstag der Anmeldung: 15.05.1991
(73) Patentinhaber: BASF Aktiengesellschaft, 67063 Ludwigshafen (DE)
(72) Erfinder: SCHÖLLKOPF, Ulrich, D-3406 Bovenden (DE); GROTH, Ulrich, D-3400 Göttingen (DE); LANGE, Meinolf, D-3401 Waake/Bösinghausen (DE)
(86) Internationale Anmeldenummer: EP8901050
(87) Internationale Veröffentlichungsnummer: WO9003386

(56) Entgegenhaltungen:
- Chemcial Abstracts, vol. 108, No. 9, 29 February 1988, (Columbus, Ohio, US), U. Schoellkopf et al.: "Asymmetric syntheses via heterocyclic intermediates, XXXVI. Asymmetric synthesis of dimethyl (R)-4-amino-4methyl-2-pentendioates (dimethyl beta,upsilon-didehydro-alpha-methyl-D-glutamates) or methyl (R)-2,5-dihydro-2-methyl-5-oxo-2-pyrrolecarboxylates by the bislactim ether method. Studies on the asymmetric synthesis of beta,upsilon-didehydroglutamic acids", page 726, abstract 75801u
- Chemical Abstracts, vol. 109, No. 17, 24 October 1988, (Columbus, Ohio, US), U. Schoellkopf et al.: "Asymmetric syntheses via heterocyclic intermediates. XL. Studies on the acylation of lithiated bislactim ethers of cyclo-(L-Val-Ala-) and cyclo (L-Val-Gly-)", see page 785, abstract 150014r
- Tetrahedron, vol. 39, No. 12, 1983, Pergamon Press, (Oxford, GB), U. Schöllkopf: "Enantioselective synthesis of non-proteinogenic amino acids via metallated bis-lactim ethers of 2, 5-diketopiperazines", pages 2085-2091 see the whole article.
- Liebigs Annalen der Chemie, issue 11, November 1988, VCH Verlagsgesellschaft mbH, (Weinheim, DE), U. Schöllkopf et al.: "Asymmetric synthesis of Boc-L-Val-(R)- -MePro-OMe, Boc-L-Val-(R)-Pro-OMe, and of Boc-L-Val-(R)- -MePHe-OMe, Ac-L-Val-(R)- -MePHe-OMe and their analogues. A new strategy for the synthsis of non-proteinogenic bipeptides", pages 1025-1031, see the whole article in particular pages 1025-1027 and the production of compounds 17 and 28, 28b and 28c of pages 1029-1031.

## Beschreibung

Die Vorliegende Erfindung betrifft ein Verfahren zur Herstellung von Dipeptiden, die sich von nicht-proteinogenen Aminosäuren ableiten.

Zur gezielten Synthese von Peptiden, die nicht proteinogene Aminosäuren enthalten, werden die unnatürlichen Aminosäuren unter Verwendung üblicher Schutzgruppen und Aktivierungsmethoden zum stufenweisen Aufbau der gewünschten Peptide eingesetzt.

Die eingesetzten unnatürlichen Aminosäuren werden bei dieser Strategie entweder über eine nicht enantioselektive Synthese und anschließende Racematspaltung oder mittels enantioselektiver Synthesen unter Verwendung optisch aktiver Hilfsstoffe gewonnen.

Schwierigkeiten bei der Ausbildung der Peptidbindung können besonders dann auftreten, wenn die α-Position der unnatürlichen Aminosäure durch zwei sterisch anspruchsvolle Reste substituiert ist (Helv. Chim. Acta 69, (1986) 1153, J. Amer. Chem. Soc. 103 (1981) 6127).

Es wurde nun gefunden, daß man bestimmte Dipeptide auf einfache Weise herstellen kann.

Gegenstand der Erfindung ist ein Verfahren zur Herstellung von Dipeptiden mit C-terminalen nicht-proteinogenen Aminosäuren der Formel I 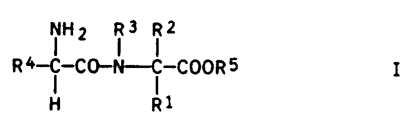
worin
- R¹: eine C₁-C₈-Alkyl-, Phenyl- oder Benzylgruppe ist
- R²: eine C₁-C₈-Alkylgruppe, die durch -O-, -S-, -CO- oder -CO-O-unterbrochen sein kann, oder eine Phenyl- oder Benzylgruppe bedeutet,
- R³: ein Wasserstoffatom oder eine C₁-C₈-Alkylgruppe ist oder zusammen mit R² die Reste -(CH₂)₃-, -(CH₂)₄- oder -CH₂-CH=CH-CH₂- darstellt und
- R⁴: einen Methyl-, Isopropyl-, Isobutyl-, 2-Butyl-, t-Butyl- oder Benzylrest bedeutet,
- R⁵: eine Methyl- oder Ethylgruppe darstellt,
welches darin besteht, daß man eine Verbindung der Formel II 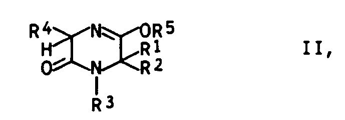
worin R¹ bis R⁵ die angegebene Bedeutung haben, hydrolysiert.

Die Hydrolyse der Verbindungen II zu den Endprodukten gelingt besonders gut in verdünnten wäßrigen Mineralsäuren in Gegenwart von organischen Kosolventien wie Methanol, Ethanol, Tetrahydrofuran, Acetonitril oder Dioxan. Als Mineralsäuren werden vorzugsweise HCl, HBr und HI verwendet. Die Umsetzung wird in der Regel bei 0 bis 40°C durchgeführt. Die Reaktionsdauer beträgt 15 min bis 6 h.

Die Verbindungen II lassen sich durch eine sauer katalysierte, selektive Partialspaltung aus den Verbindungen III 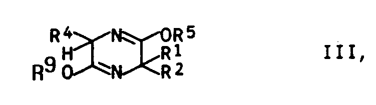
worin R¹, R², R⁴ and R⁵ die angegebene Bedeutung besitzen und R⁹ eine Methyl- oder Ethylgruppe darstellt, erhalten. Die sauer katalysierte, selektive Partialspaltung wird so ausgeführt, daß man die Verbindungen III mit einem Äquivalent Säure in einem aprotischen, inerten Lösungsmittel umsetzt. Als Säuren sind insbesondere HCl, HBr und HI sowie Lewis-Säuren wie Trialkylsilylhalogenide oder Dialkoxyborhalogenide zu nennen. Geeignete Lösungsmittel sind Diethylether, Dibutylether, Tetrahydrofuran, Dioxan, Dichlormethan, Tetrachlorkohlenstoff, Toluol, Cyclohexan und Ethylenglykoldimethylether. Die Umsetzung wird in der Regel bei 0 bis 40°C durchgeführt. Die Reaktionsdauer beträgt 15 min bis 6 h.

Die Verbindungen II, in denen R³ Wasserstoff ist, lassen sich durch baseninduzierte Alkylierung mit Alkylhalogeniden oder Alkylsulfaten in die Verbindungen II überführen, in denen R³ eine C₁-C₈-Alkylgruppe ist.

Die Verbindungen der Formel II, in denen R³ ein Wasserstoffatom und R⁵ eine Ethylgruppe sind, lassen sich besonders gut aus den Verbindungen der Formel IV 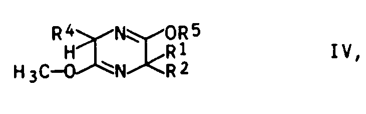
durch Demethylierung mit einem Trialkylsilyliodid mit 1-4 C-Atomen in den Alkylresten, darstellen. Die Umsetzung wird zweckmäßigerweise bei 0 bis -20°C in einem wasserfreien Halogenkohlenwasserstoff als Lösungsmittel und unter einem Inertgas durchgeführt. Als Trialkylsilyliodide werden vorzugsweise solche der Formel 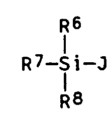
verwendet, worin R⁶-R⁸ C₁₋₄-Alkylreste sind.

Da die Verbindungen I als freie Basen unter Ringschluß teilweise leicht die entsprechenden Diketopiperazine bilden, werden die bei der Hydrolyse anfallenden Salze von I vorzugsweise entweder durch Umsetzung mit den in der Peptidchemie üblichen Acylierungsmitteln wie Benzoyloxycarbonylchlorid, Fluorenyl-9-methoxycarbonylchlorid oder Di-tert.-Butylcarbonat in die N-geschützten Derivate überführt (vgl. Houben-Weyl, Methoden der organischen Chemie, Bd. 15/1, G. Thieme Verlag, Stuttgart 1974), oder mit aktivierten Aminosäurederivaten (vgl. Houben-Weyl, Methoden der organischen Chemie, Bd. 15/2, G. Thieme Verlag, Stuttgart 1974) zu Tripeptidderivaten umgesetzt.

Die Verbindungen I besitzen zwei asymmetrische C-Atome, die (R)- und (S)-konfiguriert sein können und deren Konfiguration gleich oder verschieden sein kann.

Die für die Synthese der Dipeptide benötigten Ausgangsstoffe III sind bekannt, vgl. Pure Appl. Chem. 55, 1799 (1983), Angew. Chem. 99, 137 (1987) und frühere Publikationen dieser Serie.

Die Verbindungen IV, in denen R⁵ eine Ethylgruppe darstellt, lassen sich besonders einfach herstellen, indem man eine Verbindung der Formel V 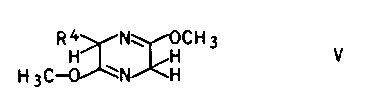
mit einem Mono- oder Dialkylamin in Gegenwart eines Borsäuretrialkylesters oder einer anderen milden Lewis-Säure zu einer Verbindung der Formel VI 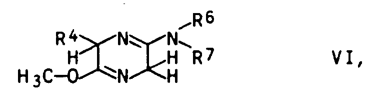
worin R⁶ ein Wasserstoffatom oder eine C₁-C₆-Alkylgruppe und R⁷ eine C₁-C₆-Alkylgruppe darstellen, umsetzt, den Aminrest hydrolytisch entfernt und das Hydrolyseprodukt anschließend mit Triethyloxoniumtetrafluoborat in der 6-Stellung ethyliert. Die so erhaltenen gemischten Bislactimether der Formel VII 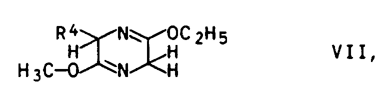
(VII = IV, R¹ = R² = H, R⁵ = C₂H₅) können anschließend durch einfache oder doppelte C-Alkylierung ihrer Lithiumsalze in die Zwischenprodukte IV überführt werden.

Die Umsetzung von V zu VI erfolgt bevorzugt durch Reaktion von V mit einer methanolischen Lösung von etwa 1,5 Äquivalenten Mono- oder Dialkylamin in Anwesenheit katalytischer Mengen einer Lewis-Säure bei 20 bis 60°C. Die Verbindungen VI werden durch Destillation gereinigt.

Die Umsetzung von VI zu VII verläuft in zwei Schritten. Die Aminogruppe wird im ersten Schritt durch Hydrolyse abgespalten. Hierzu wird VI in Wasser bei pH 6 bis 9 bei 50 bis 100°C gerührt. Anschließend wird mit einem Lösungsmittel wie Halogenkohlenwasserstoff oder Essigsäureethylester extrahiert und der erhaltene Monolactimether mit Triethyloxoniumtetraborat alkyliert. Die Alkylierung wird in einem Chlorkohlenwasserstoff bei Raumtemperatur durchgeführt und dauert in der Regel 1 bis 3 Tage. Im Anschluß an die Alkylierung wird die Lösung neutralisiert und die Verbindung VII extrahiert.

Das erfindungsgemäße Verfahren zur Herstellung der α-alkylverzweigten Dipeptide zeichnet sich dadurch aus, daß der Aufbau der unnatürlichen Aminosäure an einem ohne Schwierigkeiten zugänglichen Lactimetherderivat, d.h. einem maskierten Dipeptidderivat, erfolgt. Dieser chirale "Hilfsstoff" II wird Bestandteil des gewünschten Dipeptids. Durch die Erfindung wird es möglich, in den Verbindungen III bzw. IV nur eine Etherbindung zu spalten. So entsteht aus III mit einem Äquivalent Säure überraschenderweise ganz überwiegend der Monolactimether II. Eventuell entstehende Regioisomere werden durch Chromatographie oder Destillation abgetrennt. In besonders einfacher Weise lassen sich Verbindungen der Formel II durch selektive Demethylierung mit Trialkylsilyliodid aus IV gewinnen. Durch selektive Hydrolyse mit wäßriger Säure erhält man von II ausgehend I.

Verbindungen der Formel I sowie die daraus in einfacher Weise durch Umsetzung mit den üblichen Acylierungsmitteln wie Benzyloxycarbonylchlorid, Fluorenyl-9-methoxycarbonylchlorid oder Di-tert.-Butyldicarbonat ((BOC)₂O) herstellbaren N-geschützten Derivate dienen als Bausteine zum Aufbau biologisch aktiver Peptide. Das Verfahren eignet sich zur Herstellung von Bausteinen für oral wirksame Peptide bzw. Peptid-Analoga, die das Enzym Renin hemmen und daher für die Therapie des Bluthochdrucks eingesetzt werden können.

### Beispiele

1. BOC-L-Val-R-α MePhe-OMe
1,44 g (2S,5R)-2,5-Dihydro-2-isopropyl-3,6-dimethoxy-5-benzyl-5-methyl-pyrazin in 30 ml Diethylether wurden bei Raumtemperatur mit 25 ml 0,2 N HCl in Diethylether behandelt. Nach 2 h wurde im Vakuum eingedampft. Destillation des Rückstands gab 0,94 g (69 %) rohes (2S,5R)-2,5-Dihydro-2-isopropyl-6-methoxy-5-benzyl-5-methyl-pyrazin-3-on, Kp. 170°C/0,005 Torr.
Zu einer Lösung von 0,35 g des oben erhaltenen Pyrazinons in 50 ml Acetonitril wurden 50 ml 0,1 N HCl zugegeben. Unter Rühren wurde 24 h bei Raumtemperatur umgesetzt. Zur Aufarbeitung wurde zunächst nicht umgesetztes Pyrazinon durch Extraktion mit 3 x 30 ml Dichlormethan entfernt. Die wäßrige Phase wurde mit Ammoniak auf pH 9 gebracht und mit Dichlormethan extrahiert (3 x 10 ml). Der organische Extrakt wurde über Magnesiumsulfat getrocknet und im Vakuum zur Trockne eingedampft. Man erhielt 0,3 g rohes H-Val-R-αMePhe-OMe welches sofort nach dem üblichen Verfahren mit Di-tert.-Butyldicarbonat/Triethylamin in Dichlormethan in die BOC-Verbindung überführt wurde. Nach Reinigung durch Chromatographie an Kieselgel (Elution mit Ether/Petrolether 1:3) wurden 0,35 g (90 %) BOC-L-Val-R-αMePhe-OMe, Schmp. 57°C, erhalten.
2. BOC-L-Val-R-αMePro-OMe
0,45 g (2S,5R)-2,5-Dihydro-2-isopropyl-5-methyl-6-methoxy-4,5-pyrrolidinopyrazin-3-on in 20 ml Tetrahydrofuran wurden bei Raumtemperatur mit 30 ml 3N HCl versetzt. Eindampfen zur Trockne und Umsetzung mit Di-tert.-Butyldicarbonat in Triethylamin ergab nach Reinigung durch Chromatographie an Kieselgel (Elution mit Ether/Petrolether 1:1) 0,43 g BOC-L-Val-R-αMePro-OMe, Schmp. 92-95°C.
Analog wurden erhalten:
3. BOC-L-Val-R-αMe-Pipecolinsäure-OMe; Schmp. 81-85°C;
4. BOC-L-Val-R-αMe-Baikiainsäure-OMe; Schmp. 31-35°C.
5. BOC-L-Val-(D)-N-methyl-Me-Phe-OEt
a) 0,3 g (2R, 5S)-2-Benzyl-2,5-dihydro-3-ethoxy-5-isopropy-6-methoxy-2-methyl-pyrazin wurden in 15 ml absoluten Dichlormethan gelöst und unter Argon auf -10°C abgekühlt. Anschließend wurde 25 mg Iod und 0,16 ml Trimethylsilyliodid hinzugegeben. Nach 3 h ließ man die Lösung auf Raumtemperatur erwärmen und rührte 14 h bei 22°C. Nach Zugabe von 5 ml Ethanol und 5 ml Diethylamin wurde im Vakuum eingeengt und der Rückstand über Kieselgel (Ether, Rf=0,44) chromatographiert. Man erhielt 0,29 g (91 %) (2R, 5S)-2-Benzyl-3-ethoxy-5-isopropyl-1,2,5,6-tetrahydro-2-methyl-pyrazin-6-on, Schmp 125°C.
b) 0,25 g des gemäß a) erhaltenen Produkts wurden in 10 ml Tetrahydrofuran gelöst und nach Zugabe von 7 mg Natriumhydrid 1,5 h bei Raumtemperatur gerührt. Nach Zugabe von 0,5 ml Dimethylsulfat wurde weitere 10 h gerührt. Anschließend wurden 2 ml Dimethylamin und 4 ml Ethanol zugegeben und das Lösungsmittel abgezogen. Man erhielt nach Chromatographie an Kieselgel (Diethylether/Petrolether 1:1, Rf=0,14) 0,25 g (96 %) (2R, 5S)-2-Benzyl-3-ethoxy-5-isopropyl-1,2,5,6-tetrahydro-2-methyl-pyrazin-6-on.
c) 0,2 g des gemäß b) erhaltenen Produkts wurden in 10 ml Acetonitril gelöst und mit 2,7 ml 1 N Salzsäure versetzt. Nach 5 h stehen bei Raumtemperatur wurde im Vakuum eingeengt. Anschließend wurde in 1,2 ml Triethylamin und 10 ml Dichlormethan gelöst und mit 0,3 g BOC-Anhydrid versetzt. Das Gemisch wurde 12 h bei Raumtemperatur gerührt und danach im Vakuum eingeengt. Der Rückstand wurde an Kieselgel chrometographisch (Petrolether/Diethylether 3:1, Rf=0,11). Man erhielt 0,26 g (85 %) Produkt.

| | | | |
|---|---|---|---|
| C₂₃H₃₆N₂O₅ (420,55) | Ber. | C = 65,69 | H = 8,63 |
| | Gef. | C = 65,51 | H = 8,60 |

6. (L)-N-BOC-Val-(D)-Me-Phe-OEt
Das Beispiel wurde ohne den Verfahrensschritt b) wiederholt. So wurde das Endprodukt in 83 %iger Ausbeute erhalten, Schmp 102°C.

## Patentansprüche

1. Verfahren zur Herstellung von Dipeptiden mit C-terminalen nicht-proteinogenen Aminosäuren der Formel I 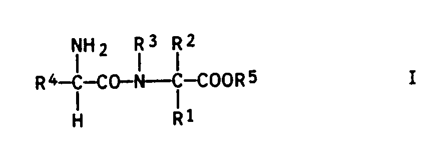 worin
R¹ eine C₁-C₈-Alkyl-, Phenyl- oder Benzylgruppe ist
R² eine C₁-C₈-Alkylgruppe, die durch -O-, -S-, -CO- oder -CO-O- unterbrochen sein kann, oder eine Phenyl- oder Benzylgruppe bedeutet,
R³ ein Wasserstoffatom oder eine C₁-C₈-Alkylgruppe ist oder zusammen mit R² die Reste -(CH₂)₃-, -(CH₂)₄- oder -CH₂-CH=CH-CH₂-darstellt und
R⁴ einen Methyl-, Isopropyl-, Isobutyl-, 2-Butyl-, t-Butyl- oder Benzylrest bedeutet,
R⁵ eine Methyl- oder Ethylgruppe darstellt,
dadurch gekennzeichnet, daß man eine Verbindung der Formel II 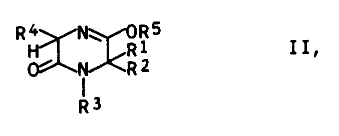 worin R¹ bis R⁵ die angegebene Bedeutung haben, hydrolysiert.

2. Verfahren gemäß Anspruch 1, dadurch gekennzeichnet, daß man die Verbindungen der Formel II, in denen R³ Wasserstoff oder einen C₁₋C₈-Alkylrest bedeutet, durch selektive Partialspaltung von Verbindungen der Formel III 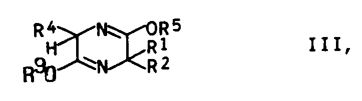 worin R¹, R², R⁴ und R⁵ die in Anspruch 1 angegebene Bedeutung haben, und R⁹ eine Methyl- oder Ethylgruppe darstellt, und gegebenenfalls anschließende N-Alkylierung erhält.

3. Verfahren gemäß Anspruch 1, dadurch gekennzeichnet, daß man die Verbindungen der Formel II (R³=H) durch Demethylierung von Verbindungen der Formel IV 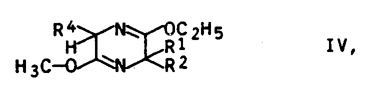 worin R¹, R² und R⁴ die in Anspruch 1 angegebene Bedeutung besitzen, mit einem Trialkylsiliciumiodid erhält.

## Claims

1. A process for preparing dipeptides with C-terminal non-proteinogenous amino acids of the formula I 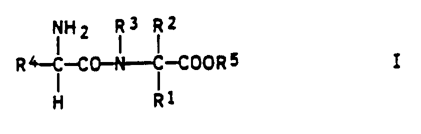 where
R¹ is C₁-C₈-alkyl, phenyl or benzyl
R² is C₁-C₈-alkyl which can be interrupted by -O-, -S-, -CO- or -CO-O-, or is phenyl or benzyl,
R³ is hydrogen or C₁-C₈-alkyl or, together with R², the radicals -(CH₂)₃-, -(CH₂)₄- or -CH₂-CH=CH-CH₂- and
R⁴ is methyl, isopropyl, isobutyl, 2-butyl, t-butyl or benzyl,
R⁵ is methyl or ethyl,
which comprises hydrolyzing a compound of the formula II 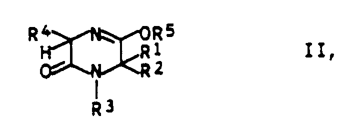 where R¹ to R⁵ have the stated meanings.

2. A process as claimed in claim 1, wherein the compounds of the formula II where R³ is hydrogen or C₁-C₈-alkyl are obtained by selective partial cleavage of compounds of the formula III 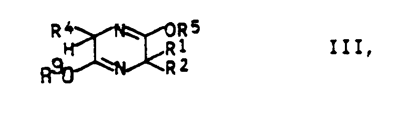 where R¹, R², R⁴ and R⁵ have the meanings stated in claim 1, and R⁹ is methyl or ethyl and, where appropriate, subsequent N-alkylation.

3. A process as claimed in claim 1, wherein the compounds of the formula II (R³=H) are obtained by demethylation of compounds of the formula IV 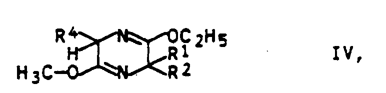 where R¹, R² and R⁴ have the meanings stated in claim 1, with a trialkylsilicon iodide.

## Revendications

1. Procédé de préparation de dipeptides avec des aminoacides non protéinogènes C-terminaux de la formule 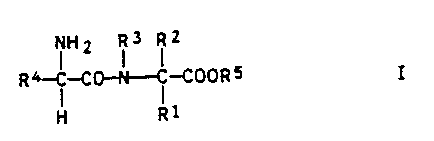 dans laquelle
R¹ représente un radical alkyle en C₁-C₈, phényle ou benzyle,
R² représente un radical alkyle en C₁-C₈, qui peut être interrompu par -O-, -S-, -CO- ou -CO-O-, ou un groupe phényle ou benzyle,
R³ représente un atome d'hydrogène ou un radical alkyle en C₁-C₈, ou forme, ensemble avec R², les restes -(CH₂)₃-, -(CH₂)₄- ou -CH₂-CH=CH-CH₂- et
R⁴ représente un radical méthyle, isopropyle, isobutyle, 2-butyle, t-butyle ou benzyle.
R⁵ représente un groupe méthyle ou éthyle,
caractérisé en ce que l'on hydrolyse un composé de la formule II 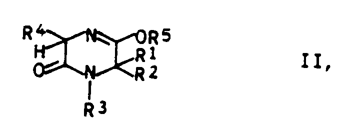 dans laquelle R¹ à R⁵ possèdent les significations qui leur ont été attribuées ci-dessus.

2. Procédé suivant la revendication 1, caractérisé en ce que l'on obtient les composés de la formule II dans laquelle R³ représente un atome d'hydrogène ou un radical alkyle en C₁-C₈, par la scission partielle sélective de composés de la formule III 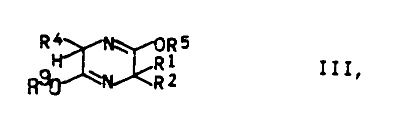 dans laquelle R¹, R², R⁴ et R⁵ possèdent les significations qui leur ont été attribuées dans la revendication 1 et R⁹ représente un groupe méthyle ou éthyle et N-alkylation subséquente éventuelle.

3. Procédé suivant la revendication 1, caractérisé en ce que l'on obtient les composés de la formule II (R³ = H) par la déméthylation de composés de la formule IV 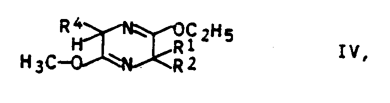 dans laquelle R¹, R² et R⁴ possèdent les significations qui leur ont été attribuées dans la revendication 1, avec un iodure de trialkylsilicium.
